# FASCICULE DE BREVET EUROPEEN

(11) **EP 2 688 681 B1**
(45) Date de publication et mention de la délivrance du brevet: **11.02.2015**
(21) Numéro de dépôt: 12720225.7
(22) Date de dépôt: 19.03.2012
(51) Int. Cl.: B05B 11/00, B65D 83/38, A61M 11/02, A61M 15/08, B65D 83/22, A61M 15/00, B65D 83/20

(54) **DISPOSITIF DE DISTRIBUTION DE PRODUIT FLUIDE A ACTIONNEMENT LATERAL**
SEITLICH BETÄTIGTE VORRICHTUNG ZUR AUSGABE EINES FLÜSSIGPRODUKTS
LATERALLY-ACTUATED FLUID PRODUCT DISPENSING DEVICE

(30) Priorité: 21.03.2011 FR 1152301
(43) Date de publication de la demande: 29.01.2014
(73) Titulaire: Aptar France SAS, 27110 Le Neubourg (FR)
(72) Inventeur: PARDONGE, Jean-Marc, F-76520 Les Authieux Sur Port Saint Ouen (FR)
(74) Mandataire: CAPRI
(86) Numéro de dépôt international: PCT/FR2012/050573
(87) Numéro de publication internationale: WO 2012/127166

(56) Documents cités:
- EP-A2- 1 407 826
- WO-A1-2006/097747
- WO-A1-2009/068877
- WO-A1-2009/153513
- DE-A1- 19 610 456
- FR-A- 1 355 588
- FR-A1- 2 812 826
- FR-A1- 2 832 329
- FR-A1- 2 882 349
- US-A1- 2002 170 928

## Description

La présente invention concerne un dispositif de distribution de produit fluide à actionnement latéral, et plus particulièrement un dispositif de pulvérisation nasale d'un produit pharmaceutique.

Les dispositifs de distribution de produit fluide sont bien connus dans l'état de la technique. Ils comportent généralement un réservoir contenant le produit fluide sur lequel est assemblé un organe de distribution, par exemple une pompe ou une valve, qui est généralement actionné au moyen d'une tête de distribution pour distribuer de manière sélective le produit contenu à l'intérieur dudit réservoir. La tête de distribution comporte un orifice de distribution à travers lequel le produit va être pulvérisé, par exemple dans le nez de l'utilisateur dans le cas d'un dispositif de pulvérisation nasale. De nombreux dispositifs de ce type sont actionnés manuellement par l'utilisateur en déplaçant axialement l'un contre l'autre le réservoir et la tête de distribution, ce qui a pour effet d'actionner l'organe de distribution. Ce type de dispositif présente toutefois des inconvénients, notamment lorsque le dispositif est du type à pulvérisation nasale, car la force axiale exercée par l'utilisateur pour actionner le dispositif induit un risque de déplacement de la tête de distribution à l'intérieur de la narine de l'utilisateur, avec des risques de blessure et/ou de distribution incomplète ou inadéquate du produit au moment de l'actionnement. Pour remédier à ce problème, des dispositifs d'actionnement latéral ont été proposés, comportant généralement un levier monté pivotant sur un corps et dont une partie interne est adaptée à coopérer avec l'un parmi la tête de distribution ou le réservoir pour déplacer cet élément contre l'autre et ainsi actionner l'organe de distribution. Ces dispositifs d'actionnement latéral présentent toutefois un certain nombre de problèmes. Ainsi, ils induisent généralement des contraintes radiales lors de l'actionnement qui peuvent aussi avoir des influences négatives sur la pulvérisation du produit dans la narine de l'utilisateur. Ils peuvent présenter des problèmes de fiabilité lors de l'actionnement, avec dans certains cas des risques de blocages susceptibles d'empêcher tout actionnement. Ils comportent généralement des pièces mobiles qui imposent une direction d'actionnement bien définie, avec des risques de dysfonctionnement en cas d'actionnement selon une direction un peu décalée. Ils sont susceptibles de générer des bruits lors de l'actionnement ou si le dispositif est secoué, ce qui peut être perçu négativement par l'utilisateur. Ils sont souvent assez complexes à assembler, et cet assemblage n'est généralement possible qu'après remplissage du produit à distribuer, ce qui impose des lignes de montages complexes chez le fabricant de produit actif, qui est généralement différent du fabricant du dispositif d'actionnement latéral. Les documents WO 2005/075105, WO 2009/153513, WO 2009/068877, US 2002/170928, FR-2 832 329, FR-2 812 826, EP-1 407 826, FR-1 355 588, FR-2 882 349, WO 2006/097747 et DE 196 10 456 décrivent des dispositifs de l'art antérieur. Ces documents décrivent des systèmes d'actionnement latéral qui poussent soit sous un bord inférieur d'une bague de fixation ou d'une bague rapportée, soit sous un profil radial formé sur une telle bague.

Le document FR-2 832 329 décrit un dispositif selon le préambule de la revendication 1.

La présente invention a pour but de fournir un dispositif de distribution de produit fluide à actionnement latéral qui ne reproduit pas les inconvénients susmentionnés.

La présente invention a aussi pour but de fournir un dispositif de distribution de produit fluide à actionnement latéral, notamment de pulvérisation nasale, qui garantit un actionnement sûr et fiable du dispositif à chaque actionnement, sans risque de blessure pour l'utilisateur.

Plus particulièrement, la présente invention a pour but de fournir un dispositif de distribution de produit fluide qui soit simple et peu coûteux à fabriquer et à assembler.

La présente invention a donc pour objet un dispositif de distribution de produit fluide, comportant un corps, un réservoir, une tête de distribution incorporant un orifice de distribution, un organe de distribution, tel qu'une pompe ou une valve, monté sur ledit réservoir par une bague de fixation, ledit organe de distribution comportant un corps d'organe de distribution et un organe mobile déplacé axialement dans ledit corps d'organe de distribution lors de l'actionnement dudit organe de distribution, ledit dispositif comportant en outre un système d'actionnement latéral solidaire dudit corps, comportant un élément d'actionnement coopérant avec ladite bague de fixation, ledit élément d'actionnement étant mobile, sensiblement transversalement à la direction de déplacement dudit organe mobile, entre une position de repos, dans laquelle ledit organe de distribution n'est pas actionné, et une positionnement d'actionnement, dans laquelle ledit organe de distribution est actionné, ladite bague de fixation comportant une bride radiale supérieure s'étendant radialement vers l'extérieur, ledit élément d'actionnement comportant au moins une projection radiale dont un bord supérieur vient en contact avec un bord inférieur de ladite bride radiale lors de l'actionnement.

Avantageusement, ledit corps comporte au moins une surface de guidage dudit élément d'actionnement, ladite au moins une surface de guidage étant fixe par rapport à ladite tête de distribution et sensiblement parallèle à l'axe de déplacement de l'organe mobile dans le corps d'organe de distribution lors de l'actionnement dudit organe de distribution, ledit élément d'actionnement comportant au moins un bord d'extrémité, de préférence arrondi, coopérant avec ladite au moins une surface de guidage.

Avantageusement, ledit élément d'actionnement comporte au moins un flanc s'étendant sensiblement depuis l'axe de rotation dudit élément d'actionnement jusqu'à ladite surface de guidage, le bord d'extrémité dudit flanc étant arrondi et glissant sur ladite surface de guidage lors de l'actionnement, chaque flanc comportant une projection radiale saillante s'étendant sur un coté dudit flanc, à proximité de son bord d'extrémité, ladite projection radiale saillante ayant un bord supérieur, de préférence arrondi, coopérant avec ladite bride radiale de ladite bague de fixation.

Avantageusement, ledit élément d'actionnement comporte deux flancs s'étendant parallèlement l'un à l'autre, lesdites projections radiales saillantes se faisant face.

Avantageusement, ladite bague de fixation comporte une première partie de fixation qui coopère avec le réservoir pour assurer la fixation de ladite bague de fixation sur ledit réservoir, une seconde partie qui coopère avec l'organe de distribution pour assurer la fixation dudit organe de distribution dans ladite bague de fixation, et une troisième partie comportant ladite bride radiale supérieure.

Avantageusement, ladite bride radiale supérieure est reliée à un manchon axial, ladite au moins une projection radiale de l'élément d'actionnement étant disposée à l'extérieur dudit manchon axial, avec le bord supérieur de ladite projection radiale en contact avec ladite bride radiale lors de l'actionnement.

Avantageusement, ledit système d'actionnement latéral comporte un élément d'appui monté pivotant autour d'un axe A sur le corps, ledit élément d'actionnement étant monté pivotant sur ledit élément d'appui autour d'un axe B.

Avantageusement, ledit élément d'actionnement comporte un élément élastique, tel qu'une lame élastique, coopérant avec ledit élément d'appui pour solliciter ledit élément d'actionnement en éloignement dudit élément d'appui.

Ces caractéristiques et avantages et d'autres de la présente invention apparaîtront plus clairement au cours de la description détaillée suivante de plusieurs modes et variantes de réalisation de celle-ci, faite en référence aux dessins joints, donnés à titre d'exemples non limitatifs, sur lesquels :
- la figure 1 représente une vue schématique découpée d'un dispositif selon un mode de réalisation avantageux ;
- les figures 2 et 3 représentent des vues schématiques en coupe d'un dispositif selon un mode de réalisation avantageux, respectivement selon deux plans de coupe différents ;
- Les figures 4 et 5 représentent des vues schématiques en coupe de deux variantes de réalisation avantageuses ;
- les figures 6 et 7 représentent des vues schématiques en perspective de détails d'un mode de réalisation avantageux, respectivement dans deux position différentes ;
- la figure 8 représente une vue schématique en coupe d'un dispositif selon un mode de réalisation avantageux ;
- la figure 9 représente une vue schématique partielle d'un dispositif selon un mode de réalisation avantageux, en phase d'assemblage du dispositif ; et
- les figures 10 à 13 représentent des schémas explicatifs montrant quatre phases successives de l'assemblage, selon un mode de réalisation avantageux.

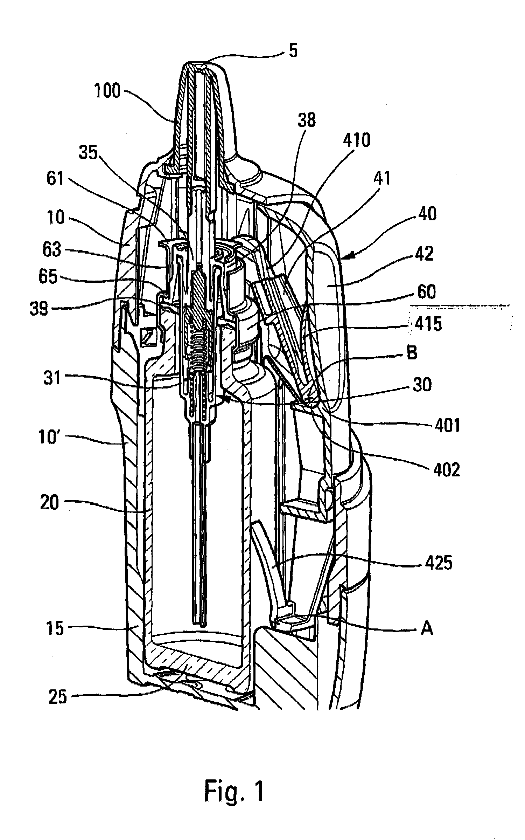

En se référant aux figures 1 à 3, qui illustrent un premier mode de réalisation avantageux, le dispositif de distribution de produit fluide comporte un corps 10 dans lequel est assemblé un réservoir 20 qui contient du produit fluide, notamment du produit pharmaceutique destiné à être pulvérisé dans le nez d'un utilisateur. Un organe de distribution 30, tel qu'une pompe ou une valve, est assemblé sur ledit réservoir 20, au moyen d'une bague de fixation 60 qui peut être sertissable, encliquetable ou vissable. Cette bague de fixation 60 peut être réalisée en métal, par exemple en aluminium, ou en matériau plastique. En variante, elle pourrait aussi être réalisée en bi-matière, avec une partie en matériau plastique et une partie en métal, typiquement par surmoulage. L'organe de distribution comporte un corps d'organe de distribution 31, tel qu'un corps de pompe ou un corps de valve, et un organe mobile 35, tel qu'une tige de piston ou une soupape de valve, qui est monté coulissant axialement dans ledit corps d'organe de distribution. De manière classique, l'organe mobile 35 est enfoncé à l'intérieur du corps d'organe de distribution 31, pour actionner ledit organe de distribution 30. Ceci est représenté schématiquement sur la figure 1, avec l'organe mobile 35, qui ici est un piston de pompe en position de repos dans un corps de pompe 31. Avantageusement, une tête de distribution 100 est assemblée sur ledit organe de distribution 30, ladite tête de distribution 100 incorporant un orifice de distribution 5 à travers lequel le produit fluide sera distribué. Dans les exemples représentés, la tête 100 est solidaire du corps 10 du dispositif ou est fixé à celui-ci. La tête 100 peut aussi être réalisée d'une pièce monobloc avec ledit corps. Le corps 10 peut être réalisé en une seule partie monobloc, ou, avantageusement, il peut comporter une partie supérieure, associée à la tête de distribution 100, et une partie inférieure 10' qui vient se fixer sur la partie supérieure lors de l'assemblage du dispositif. Ceci est représenté sur les exemples des dessins. Dans la suite de la description, il sera fait référence au corps 10, étant entendu que ceci englobe les deux variantes ci-dessus.

Le dispositif comporte un système d'actionnement latéral 40 solidaire du corps 10 et adapté à coopérer avec la bague de fixation 60 qui fixe l'organe de distribution 30 sur le réservoir 20. Ce système d'actionnement latéral 40 comporte un élément d'actionnement 41 qui est monté mobile à l'intérieur d'un élément d'appui 42, sensiblement de manière pivotante autour d'un axe B. Cet élément d'appui 42 est lui monté pivotant sur le corps 10, autour d'un axe A, sensiblement parallèle audit axe B. L'utilisateur appuyant sur l'élément d'appui 42, de préférence via une zone d'appui appropriée, pour faire pivoter cet élément d'appui 42 par rapport au corps 10 autour de l'axe A. Ce pivotement de l'élément d'appui 42 provoque le pivotement de l'élément d'actionnement 41 par rapport audit élément d'appui 42 autour de l'axe B, et ainsi l'actionnement de l'organe de distribution 30. L'élément d'actionnement 41 est donc déplaçable selon une direction sensiblement transversale à celle de l'organe mobile 35 de l'organe de distribution. Comme visible notamment sur les figures 1, 4, 5 et 8 à 13, l'élément d'actionnement 41 comporte avantageusement un élément élastique 415, tel qu'une lame élastique, qui s'appuie à l'intérieur de l'élément d'appui 42, pour solliciter élastiquement ledit élément d'actionnement 41 en éloignement dudit élément d'appui 42. Cet élément élastique pourrait être réalisé différemment, par exemple sous la forme d'un ressort quelconque. Cet élément élastique garantit que l'élément d'actionnement 41 est toujours sollicité en contact avec une surface de guidage 50, comme cela sera expliqué ci-après. Il permet aussi un assemblage sûr et fiable, comme décrit ultérieurement.

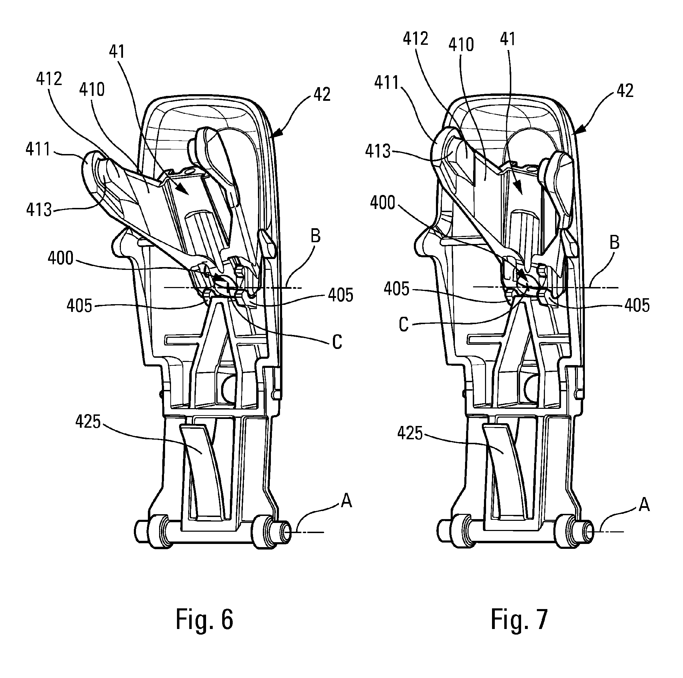

Selon un aspect avantageux, l'élément d'actionnement 41 coopère avec l'élément d'appui 42 par l'intermédiaire d'une liaison linéaire annulaire 400, ci-après désignée par rotule multidirectionnelle. Cette rotule permet un pivotement dudit élément d'actionnement 41 non seulement autour de l'axe B, mais permet également un déplacement relatif entre lesdits éléments d'actionnement 41 et d'appui 42 dans d'autres directions, en particulier autour d'un axe de pivotement C sensiblement perpendiculaire audit axe B. Elle permet aussi une certaine translation dans le sens de l'axe B. Cette rotule est notamment illustrée sur les figures 6 et 7. Cette mise en oeuvre garantir un parfait positionnement de l'élément d'actionnement 41, en compensant et/ou rattrapant les tolérances de fabrications des pièces plastiques. Cette rotule permet aussi de rattraper un mauvais actionnement de l'élément d'appui 42 par l'utilisateur, typiquement un actionnement désaxé. Ceci garantit un actionnement correct de l'organe de distribution 30 en toutes circonstances, et augmente donc la fiabilité du dispositif.

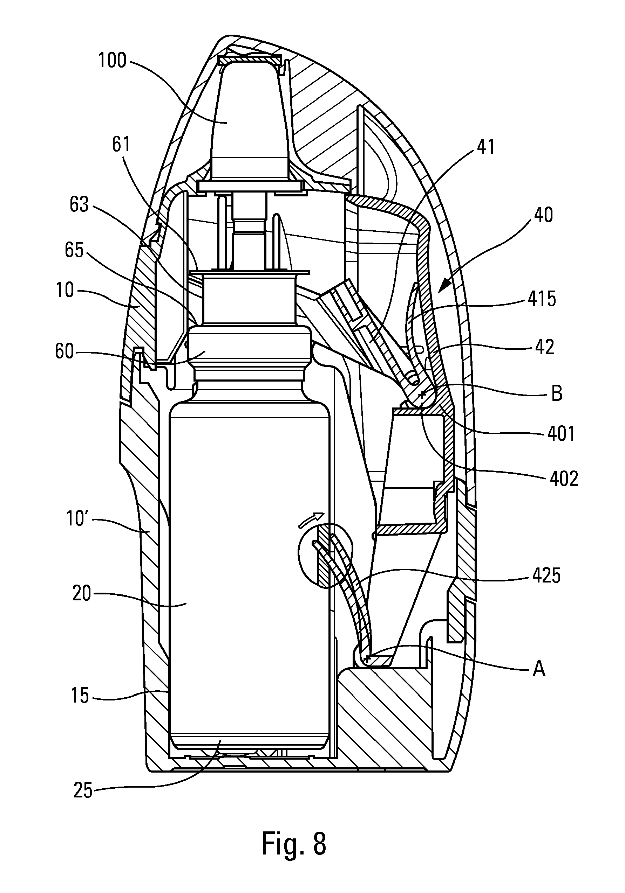

Avantageusement, comme visible notamment sur les figures 1 et 8, ladite rotule multidirectionnelle 400 peut comporter un logement creux 401 au moins partiellement arrondi formé sur ledit élément d'appui 42. L'élément d'actionnement 41 comporte alors un profil complémentaire 402 coopérant avec ledit logement creux 401, permettant un déplacement dudit élément d'actionnement 41 sur ledit élément d'appui 42 dans au moins deux directions différentes, typiquement au moins autour des axes B et C. Une certaine translation le long de l'axe B est aussi possible. Bien entendu, on peut envisager un déplacement relatif dans une infinité de directions, par exemple si le logement creux est sphérique, et que le profil complémentaire de l'élément d'actionnement est également sphérique et peut tourner dans toutes les directions à l'intérieur de ce logement sphérique. Dans une variante avantageuse, le logement creux 401 peut avoir une forme de gouttière arrondie s'étendant dans le sens de l'axe B et le profil complémentaire formé sur l'élément d'actionnement peut comporter une forme arrondie susceptible de pivoter et translater dans ladite gouttière. En variante, on peut aussi envisager de réaliser la rotule de manière inversée, avec un profil saillant partiellement arrondi, par exemple partiellement sphérique, formé sur l'élément d'appui 42, venant se positionner dans un logement creux approprié de l'élément d'actionnement 41. Dans ce cas, le logement creux serait formé sur l'élément d'actionnement et le profil complémentaire sur l'élément d'appui. D'autres variantes sont aussi envisageables.

De préférence, ladite rotule multidirectionnelle 400 comporte des moyens de butée 405 pour limiter ledit pivotement autour de l'axe de pivotement C. Comme visible sur les figures 6 et 7, ces moyens de butée 405 peuvent comporter deux supports formés sur ledit élément d'appui 42, faisant saillie de chaque coté dudit logement creux. Chaque support coopère respectivement avec une partie de l'élément d'actionnement 41 pour définir les positions d'extrémité du pivotement autour dudit axe de pivotement C, respectivement représentées sur les figures 6 et 7. Avantageusement, ces supports comportent chacun un profil supérieur arrondi adapté à guider ledit élément d'actionnement 41 vers une position relative appropriée par rapport audit élément d'appui 42. L'élément d'actionnement 41 comporte alors aussi des parties arrondies qui coopèrent avec lesdits profils arrondis des supports. Ceci permet notamment de ramener l'axe B parallèle à l'axe A, dans l'hypothèse où l'élément d'actionnement 41 aurait pu pivoter par rapport à l'élément d'appui autour d'un axe vertical sur les figures 6 et 7, provoquant un désaxement de l'un par rapport à l'autre.

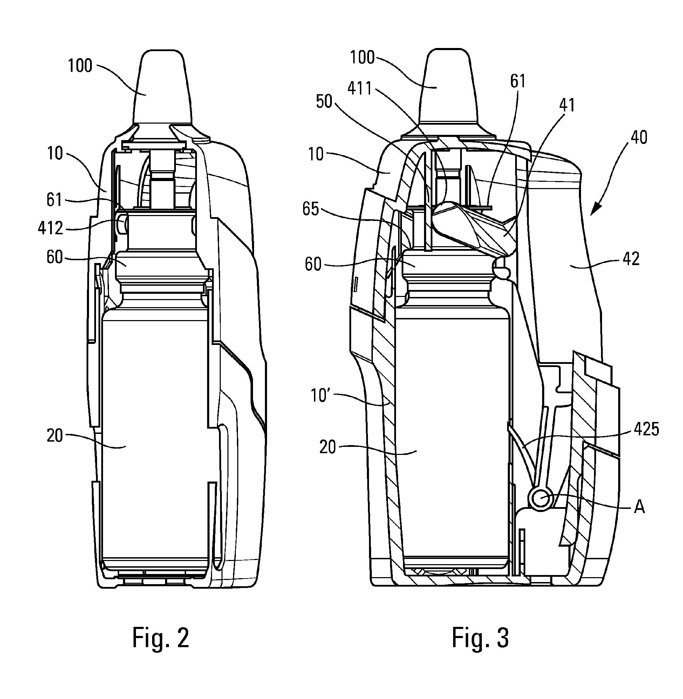

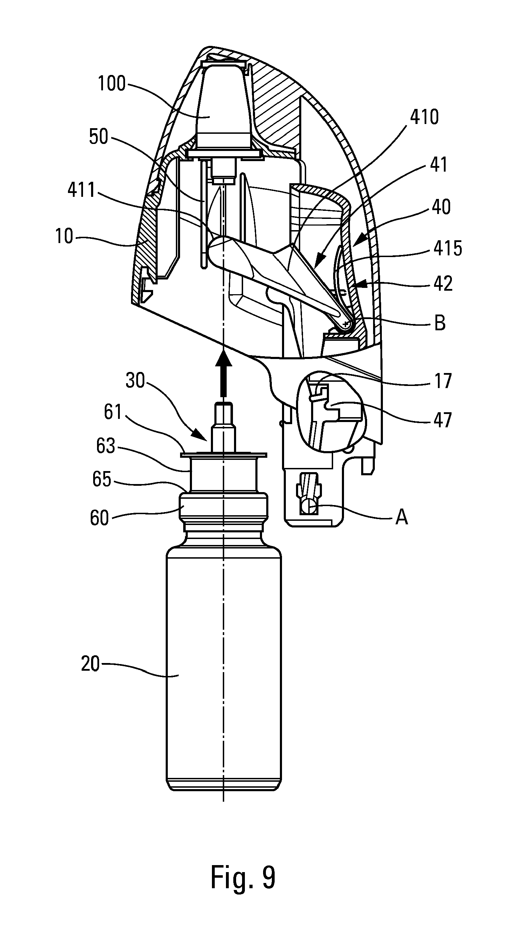

Selon un autre aspect avantageux, le corps 10 du dispositif comporte au moins une surface de guidage 50, visible en particulier sur les figures 3 à 5 et 9 à 13, qui sert à guider l'élément d'actionnement 41 pendant sa course d'actionnement, c'est-à-dire pendant son pivotement, lorsque l'utilisateur actionne le système d'actionnement latéral 40. Cette surface de guidage 50, comme visible notamment sur la figure 3, est verticale et axiale, c'est-à-dire qu'elle est sensiblement parallèle à l'axe central de l'organe de distribution, qui est l'axe de déplacement de l'organe mobile 35 dans le corps d'organe de distribution 31 lors de l'actionnement dudit organe de distribution 30. La surface de guidage 50 peut être plane, comme représentée sur la figure 3. Elle est fixe par rapport au corps 10 et à la tête de distribution 100 pendant l'actionnement. En particulier, elle peut être formée sur le corps 10, comme représenté sur la figure 3, ou sur la tête 100, comme illustré notamment sur la figure 9. Cette surface de guidage 50 sert notamment à diminuer, et notamment à supprimer, les contraintes radiales exercées par le système d'actionnement latéral 40 lors de l'actionnement du dispositif. De cette manière, il n'y a plus de risque que l'utilisateur au moment où il actionne son dispositif, déplace latéralement la tête de distribution 100 dans sa narine avec le risque que toute une partie du produit soit éjectée de manière non appropriée. Avantageusement, l'élément de l'actionnement 41 comporte au moins une zone de contact arrondie 411 pour coopérer avec cette surface de guidage 50 sur toute sa course d'actionnement. Ceci permet d'éviter au maximum les frottements contre cette surface de guidage afin de limiter au maximum l'impact de la présence de la surface de guidage 50 sur la résistance à l'actionnement du système d'actionnement latéral.

Avantageusement, la bague de fixation 60 comporte une première partie de fixation qui coopère avec le réservoir 20 pour assurer la fixation de ladite bague de fixation 60 sur ledit réservoir 20. Dans l'exemple représenté sur la figure 1, cette première partie est sertie sur le col du réservoir, mais une fixation différente, par exemple par encliquetage ou vissage, est aussi envisageable. La bague de fixation 60 comporte une seconde partie qui coopère avec l'organe de distribution 30 pour assurer la fixation dudit organe de distribution 30 dans ladite bague de fixation 60. Cette seconde partie peut par exemple comporter un épaulement 65 sensiblement radial vers l'intérieur, adapté à fixer une partie de l'organe de distribution 30 entre ledit épaulement 65 et le bord supérieur du réservoir. Dans l'exemple de la figure 1, qui montre une pompe 30 comportant un piston 35, avec une virole 38 qui définit la position de repos dudit piston, c'est une partie de ladite virole 38 et un joint de col 39 qui sont disposés dans ladite seconde partie de la bague de fixation 60. Ladite bague comporte ensuite une troisième partie comportant un manchon axial 63 pourvu à son extrémité supérieure d'une bride radiale supérieure 61 s'étendant radialement vers l'extérieur.

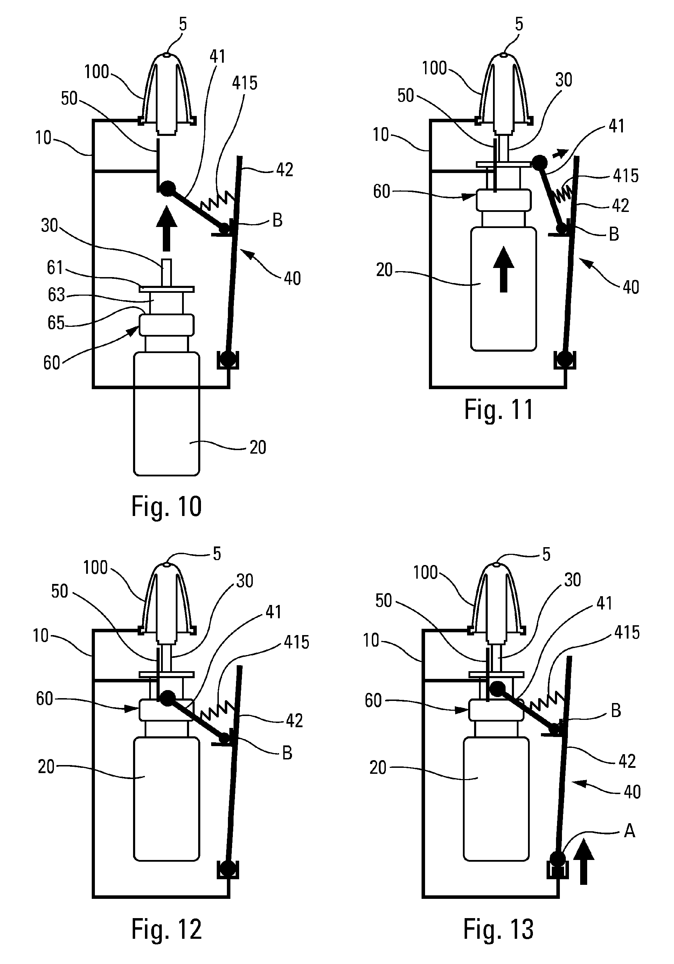

Selon un aspect avantageux, l'élément d'actionnement 41 coopère de manière opérationnelle directement avec ladite bride radiale 61 de la bague de fixation 60 lors de l'actionnement. En particulier, ledit élément d'actionnement 41 comporte avantageusement au moins un flanc 410 s'étendant sensiblement depuis l'axe de rotation B dudit élément d'actionnement 41 jusqu'à ladite surface de guidage 50. Le bord d'extrémité 411 dudit flanc 410 est de préférence arrondi et est adapté à glisser sur ladite surface de guidage 50 lors de l'actionnement. Chaque flanc 410 comporte en outre une projection radiale saillante 412 s'étendant sur un coté dudit flanc, à proximité de son bord d'extrémité. Cette projection radiale saillante 412 comporte un bord supérieur 413, de préférence arrondi, qui est adapté à coopérer avec le bord inférieur de ladite bride radiale 61 de ladite bague de fixation 60, comme notamment visible sur la figure 1. Il est à noter qu'en position de repos, il est préférable de prévoir un petit espacement entre le bord supérieur 413 de la projection radiale 412 et le bord inférieur de la bride radiale 61. Cet espacement peut être garanti par la lame élastique 415 qui sollicite l'élément d'actionnement 41 en éloignement de l'élément d'appui 42. Ce petit espacement fait qu'au tout début de l'actionnement, il n'y a pas de contact entre l'élément d'actionnement 41 et la bride radiale 61, ce contact se produisant après une petite course morte au départ de chaque actionnement. Ceci est notamment visible sur la figure 13.

Le mode de réalisation décrit notamment sur les figures 6 et 7 montre un élément d'actionnement 41 avec deux flancs 410 sensiblement parallèles l'un à l'autre, avec les projections saillantes 412 tournées vers l'intérieur et donc se faisant face. Ainsi, les deux projections radiales saillantes 412 de l'élément d'actionnement 41 sont disposées à l'extérieur dudit manchon axial 63 de la bague de fixation 60, avec le bord supérieur 413 de chaque projection radiale 412 en contact avec ladite bride radiale 61. Simultanément, les bords d'extrémité arrondis 411 des deux flancs 410 coopèrent chacun avec une surface de guidage 50 correspondante. Ceci garantit une bonne robustesse, une bonne stabilité et un fonctionnement fiable du système d'actionnement latéral. Ceci permet également de fournir un assemblage sûr et fiable de l'élément d'actionnement 41 autour de la bague de fixation 60, comme cela sera plus amplement décrit ultérieurement. Avantageusement, lesdits deux flancs 410 présentent une certaine élasticité qui leur permet de s'écarter lors de l'assemblage, et de compenser certaines tolérances de fabrication.

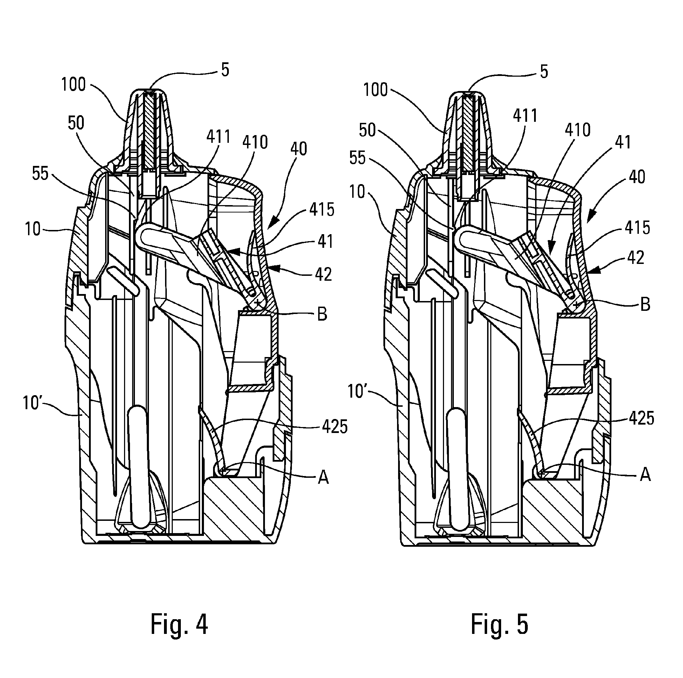

Selon un autre aspect avantageux, ladite au moins une surface de guidage 50 comporte des moyens de résistance 55 modifiant la coopération entre ledit élément d'actionnement 41 et ladite surface de guidage 50. Ainsi, lesdits moyens de résistance 55 nécessitent, pour être surmontés, une force d'actionnement différente, en particulier une force supérieure, de la part de l'utilisateur sur ledit élément d'actionnement 41. Les figures 4 et 5 illustrent deux variantes de réalisation, où la surface de guidage 50 comporte un profil saillant 55 sur la course de glissement de l'élément d'actionnement 41. Dans l'exemple de la figure 4, ce profil comporte un épaulement qui relie une première partie de surface de guidage à une seconde partie de surface de guidage. Cet épaulement est avantageusement au moins légèrement incliné, cette inclinaison pouvant être choisie en fonction de la résistance souhaitée. Dans l'exemple de la figure 4, qui représente la position de repos, on constate que ledit épaulement vient en contact avec le bord d'extrémité arrondi 411 de l'élément d'actionnement après une première partie de course d'actionnement. L'utilisateur doit alors exercer une force supérieure pour permettre audit élément d'actionnement de passer sur ledit épaulement, pour ensuite poursuivre la course d'actionnement sur la seconde partie de surface de guidage. Ceci garantit un actionnement complet, et évite les risques de mauvais actionnement de l'organe de distribution. En particulier, l'épaulement bloque l'élément d'actionnement jusqu'à ce que la force exercée par l'utilisateur soit suffisante pour surmonter cet épaulement. L'utilisateur accumule alors de l'énergie, qui sera brusquement libérée au moment où l'élément d'actionnement parviendra à passer ledit épaulement. L'exemple de la figure 5 diffère de celui de la figure 4 en ce que la surface de guidage 50 comporte une projection saillante qui coopère avec l'élément d'actionnement 41 dès le début de sa course d'actionnement. Ainsi, sur la figure 5, qui représente aussi la position de repos, l'élément d'actionnement est déjà en contact avec ladite projection. L'utilisateur doit alors exercer dès le début de l'actionnement une force suffisante pour permettre à l'élément d'actionnement de passer par-dessus ladite projection. Comme dans l'exemple de la figure 4, il accumule de l'énergie dans sa main qui sera brusquement libérée après passage de ladite projection. La course d'actionnement sera alors complète, garantissant un actionnement correct de l'organe de distribution.

D'autres variantes de réalisation des moyens de résistance sont possibles, toujours formés sur la surface de guidage 50. Par exemple, le revêtement de surface de ladite surface de guidage peut être modifié pour par exemple augmenter localement les frottements et ainsi imposer à l'utilisateur d'exercer une force d'actionnement supérieure. Ainsi, même sans profil ou projection, il est possible de modifier les conditions de coopération entre l'élément d'actionnement et la surface de guidage.

Il est avantageux de former ces moyens de résistance sur la surface de guidage, qui est fixe par rapport au corps et à la tête du dispositif, y compris pendant l'actionnement. Il est en particulier possible de prédéterminer aisément la force nécessaire pour surmonter ces moyens de résistance, et de la rendre reproductible.

Un autre avantage de ces moyens de résistance formés sur la surface de guidage est qu'ils empêchent au moins en partie un actionnement accidentel du dispositif. En effet, une certaine force est nécessaire pour surmonter ces moyens de résistance, au début ou proche du début de l'actionnement, de sorte que ces moyens de résistance procurent une certaine sécurité contre un usage non souhaité.

Selon un autre aspect avantageux, le réservoir 20 coulisse dans le corps 10 lors de l'actionnement. Ainsi, l'élément d'actionnement 41 va appuyer sous la bride radiale 61 de la bague de fixation 60, provoquant le déplacement vers le haut du réservoir 20 dans le corps 10. Ceci provoque l'actionnement de l'organe de distribution 30, en l'occurrence la pompe dans les exemples représentés. Pour assurer un maintien, notamment lors du stockage ou du transport, le corps 10 comporte avantageusement un manchon 15 adapté à être disposé au moins partiellement autour d'une partie du réservoir 20. Comme visible notamment sur les figures 1, 3 et 8, ce manchon 15 s'étend avantageusement autour de la partie inférieure du réservoir, c'est-à-dire autour du fond 25 dudit réservoir 20. Ce manchon 15 peut être annulaire, mais il est de préférence formé par une pluralité de nervures espacées les unes des autres et définissant une forme annulaire creuse. Pour ne pas risquer de bloquer ledit réservoir lors de l'actionnement, ce manchon 15 est disposé autour du réservoir avec un petit espacement. Cette mise en oeuvre présente un inconvénient en ce que la moindre secousse du dispositif provoque une oscillation ou vibration du réservoir dans ledit manchon, avec des bruits associés, du type cliquetis. D'une part, ceci peut être gênant lors de l'actionnement. D'autre part, ceci peut donner l'impression à l'utilisateur que le dispositif n'est pas solide ou mal assemblé ou de mauvaise qualité. Pour surmonter cet inconvénient, un élément élastique 425, tel qu'une lame élastique, peut être prévu, adapté à coopérer avec ledit réservoir 20 pour le solliciter contre un coté et/ou contre une nervure dudit manchon 15, comme illustré sur la figure 8. La force de la lame élastique 425 est choisie pour éviter les vibrations ou oscillations du réservoir, sans créer toutefois de fortes interactions entre ledit réservoir et ledit manchon, pour ne pas gêner l'actionnement. Le contact entre le réservoir et le manchon n'étant que limité, les frottements générés par cette interaction sont négligeables. Comme visible sur la figure 8, la lame élastique est de préférence formée sur l'élément d'appui 42 et coopère avec le réservoir 20 en dehors dudit manchon 15. Bien entendu, d'autres variantes sont envisageables. Par exemple, l'élément élastique pourrait être réalisé différemment, par exemple séparément de l'élément d'appui, et il pourrait coopérer avec le réservoir à l'intérieur du manchon.

Ainsi, la lame élastique 425, qui sollicite le réservoir contre le corps, associée à la lame élastique 415, qui sollicite l'élément d'actionnement en éloignement de l'élément d'appui, garantissent une génération de bruit minimale lors de l'actionnement et donnent à l'utilisateur une sensation de qualité et de robustesse du dispositif dans son ensemble, en éliminant substantiellement les bruits de cliquetis ou d'oscillations typiques des dispositifs pourvus de systèmes d'actionnement latéral.

Un autre aspect avantageux concerne l'assemblage du dispositif. Les figures 9 à 13 illustrent différentes étapes de l'assemblage, les figures 10 à 13 étant très schématisées pour simplifier la compréhension des différentes étapes. Comme visible sur la figure 10, le corps 10, la tête 100 et le système d'actionnement latéral 40 peuvent être pré-assemblés pour former une première unité pré-assemblée. Par ailleurs, l'organe de distribution 30 peut être fixé sur le réservoir 20 au moyen de la bague de fixation 60, formant ainsi une seconde unité assemblée. Lorsque cette seconde unité est introduite dans la première unité, comme schématisé par la flèche dans la figure 10, l'élément d'actionnement 41, qui est sollicité en contact contre la surface de guidage 50 par l'élément élastique 415, est déplacé contre cet élément élastique 415, pour permettre l'assemblage de l'organe de distribution 30 dans la tête de distribution 100, comme illustré par la figure 11. En variante, la tête 100 pourrait être assemblée sur l'unité formée par le réservoir et la pompe, puis ce sous-ensemble viendrait s'assembler dans l'unité formée par le corps et le système d'actionnement latéral.

Avantageusement, le système d'actionnement latéral 40 comporte une position pré-assemblée, dans laquelle l'élément d'appui 42 est monté sur le corps 10 dans une position pré-assemblée axialement décalée de sa position assemblée finale. La figure 9 illustre schématiquement une partie de blocage 17 du corps 10 adapté à coopérer avec une partie de pré-assemblage 47 de l'élément d'appui 42 dans cette position pré-assemblée pour bloquer le système d'actionnement latéral 40. Lorsque ledit élément d'appui 42 est déplacé de sa position pré-assemblée vers sa position assemblée, la partie de pré-assemblage 47 de l'élément d'appui 42 ne coopère plus avec ladite partie de blocage 17 du corps, venant se positionner au-dessus de cette dernière dans la position droite de la figure 9. L'axe de pivotement A de l'élément d'appui 42 vient alors prendre opérationnelle sa position de pivotement. Ainsi, dans la position de la figure 12, l'unité réservoir est assemblée dans l'unité corps/tête, mais l'élément d'appui 42 est dans sa position pré-assemblée. Le système d'actionnement latéral 40 est alors non opérationnel, et tout actionnement non souhaité reste sans effet. En particulier, la partie de blocage 17 du corps 10 bloque l'actionnement de l'élément d'appui 42 dans la position pré-assemblée. Cette mise en oeuvre fournit donc notamment une sécurité transport efficace même après assemblage de l'unité réservoir dans l'unité corps/tête. Tout actionnement du dispositif est bloqué dans cette position. Lorsque l'assemblage doit être rendu définitif, l'élément d'appui 42 est déplacé axialement vers le haut, si on se réfère à la position de la figure 13, ce qui permet à l'élément d'actionnement 41 de prendre sa position opérationnelle, sous la bride radiale 61 et à l'extérieur du manchon axial 63 de la bague de fixation 60. Le fait que l'élément élastique 415 sollicite élastiquement l'élément d'actionnement 41 en éloignement de l'élément d'appui 42 garantit un assemblage sûr et fiable dès que l'élément d'appui 42 est amené de sa position pré-assemblée à sa position assemblée. Avantageusement, le déplacement de l'élément d'appui 42 de sa position pré-assemblée vers sa position assemblée est réalisé au moment de l'assemblage de la partie inférieure 10' du corps sur le corps 10. Bien entendu, si le corps est réalisé en une seule partie, d'autres moyens peuvent être prévus pour déplacer l'élément d'appui vers sa position assemblée.

La position pré-assemblée de l'élément d'appui 42 fournit donc plusieurs avantages. D'une part, elle forme une sécurité transport pour le dispositif. De plus, elle garantit un assemblage fiable pour tout type d'organe de distribution, notamment de pompe. En effet, les pompes peuvent avoir différentes courses d'actionnement. En l'absence d'une position préassemblée, lors de l'assemblage de l'unité réservoir/pompe dans l'unité corps/tête, la pompe est actionnée pour permettre à l'élément d'actionnement 41 de venir se positionner sous la bride radiale 61 de la bague de fixation 60. Or, avec une pompe ayant une très petite course d'actionnement, il y aurait un risque que cet assemblage ne soit pas réalisé correctement. Avec l'élément d'appui 42 en position pré-assemblée au moment où l'unité réservoir/pompe est assemblée dans l'unité corps/tête, on garantit un assemblage correct lorsque l'élément d'appui 42 est déplacé de sa position pré-assemblée vers sa position assemblée. L'assemblage de l'élément d'actionnement 41 sous la bride radiale 61 n'est alors pas dépendant de la course d'actionnement de la pompe, qui peut varier selon la pompe, mais du déplacement de l'élément d'appui entre ses positions préassemblée et assemblée, qui est fixe et prédéterminé. L'assemblage correct du dispositif est donc assuré quelque soit la pompe utilisée.

Bien que l'invention ait été décrite en références à des modes de réalisations regroupant plusieurs des aspects décrits ci-dessus, d'autres modifications sont envisageables pour l'homme du métier sans sortir du cadre de la présente invention tel que défini par les revendications annexées.

## Revendications

1. Dispositif de distribution de produit fluide, comportant un corps (10), un réservoir (20), une tête de distribution (100) incorporant un orifice de distribution (5), un organe de distribution (30), tel qu'une pompe ou une valve, monté sur ledit réservoir (20) par une bague de fixation (60), ledit organe de distribution (30) comportant un corps d'organe de distribution (31) et un organe mobile (35) déplacé axialement dans ledit corps d'organe de distribution lors de l'actionnement dudit organe de distribution (30), ledit dispositif comportant en outre un système d'actionnement latéral (40) solidaire dudit corps (10), comportant un élément d'actionnement (41) coopérant avec ladite bague de fixation (60), ledit élément d'actionnement (41) étant mobile, sensiblement transversalement à la direction de déplacement dudit organe mobile (35), entre une position de repos, dans laquelle ledit organe de distribution (30) n'est pas actionné, et une positionnement d'actionnement, dans laquelle ledit organe de distribution (30) est actionné, ladite bague de fixation (60) comportant une bride radiale supérieure (61) s'étendant radialement vers l'extérieur, **caractérisé en ce que** ledit élément d'actionnement (41) comporte au moins une projection radiale (412) dont un bord supérieur (413) vient en contact avec un bord inférieur de ladite bride radiale (61) lors de l'actionnement.

2. Dispositif selon la revendication 1, dans lequel ledit corps (10) comporte au moins une surface de guidage (50) dudit élément d'actionnement (41), ladite au moins une surface de guidage (50) étant fixe par rapport à ladite tête de distribution (100) et sensiblement parallèle à l'axe de déplacement de l'organe mobile (35) dans le corps d'organe de distribution (31) lors de l'actionnement dudit organe de distribution (30), ledit élément d'actionnement (41) comportant au moins un bord d'extrémité (411), de préférence arrondi, coopérant avec ladite au moins une surface de guidage (50).

3. Dispositif selon la revendication 2, dans lequel ledit élément d'actionnement (41) comporte au moins un flanc (410) s'étendant sensiblement depuis l'axe de rotation (B) dudit élément d'actionnement (41) jusqu'à ladite surface de guidage (50), le bord d'extrémité (411) dudit flanc (410) étant arrondi et glissant sur ladite surface de guidage (50) lors de l'actionnement, chaque flanc (410) comportant une projection radiale saillante (412) s'étendant sur un coté dudit flanc (410), à proximité de son bord d'extrémité (411), ladite projection radiale saillante (412) ayant un bord supérieur (413), de préférence arrondi, coopérant avec ladite bride radiale (61) de ladite bague de fixation (60).

4. Dispositif selon la revendication 3, dans lequel ledit élément d'actionnement (41) comporte deux flancs (410) s'étendant parallèlement l'un à l'autre, lesdites projections radiales saillantes (412) se faisant face.

5. Dispositif selon l'une quelconque des revendications précédentes, dans lequel ladite bague de fixation (60) comporte une première partie de fixation qui coopère avec le réservoir (20) pour assurer la fixation de ladite bague de fixation (60) sur ledit réservoir (20), une seconde partie qui coopère avec l'organe de distribution (30) pour assurer la fixation dudit organe de distribution (30) dans ladite bague de fixation (60), et une troisième partie comportant ladite bride radiale supérieure (61).

6. Dispositif selon la revendication 5, dans lequel ladite bride radiale supérieure (61) est reliée à un manchon axial (63), ladite au moins une projection radiale (412) de l'élément d'actionnement (41) étant disposée à l'extérieur dudit manchon axial (63), avec le bord supérieur (413) de ladite projection radiale (412) en contact avec ladite bride radiale (61) lors de l'actionnement.

7. Dispositif selon l'une quelconque des revendications précédentes, dans lequel ledit système d'actionnement latéral (40) comporte un élément d'appui (42) monté pivotant autour d'un axe (A) sur le corps (10), ledit élément d'actionnement (41) étant monté pivotant sur ledit élément d'appui (42) autour d'un axe (B).

8. Dispositif selon la revendication 7, dans lequel ledit élément d'actionnement (41) comporte un élément élastique (415), tel qu'une lame élastique, coopérant avec ledit élément d'appui (42) pour solliciter ledit élément d'actionnement en éloignement dudit élément d'appui (42).

## Patentansprüche

1. Ausgabevorrichtung für ein fluides Produkt, aufweisend einen Körper (10), einen Behälter (20), einen Ausgabekopf (100), der eine Ausgabeöffnung (5) umfasst, eine Ausgabeeinrichtung (30), wie eine Pumpe oder ein Ventil, die durch einen Befestigungsring (60) auf dem Behälter (20) montiert ist, wobei die Ausgabeeinrichtung (30) einen Ausgabeeinrichtungskörper (31) und eine bewegliche Einrichtung (35) aufweist, die bei Betätigung der Ausgabeeinrichtung (30) axial in dem Ausgabeeinrichtungskörper verschiebbar ist, wobei die Vorrichtung des Weiteren ein seitliches Betätigungssystem (40) in einem Stück mit dem Körper (10) umfasst, umfassend ein Betätigungselement (41), das mit dem Befestigungsring (60) zusammenwirkt, wobei das Betätigungselement (41) im Wesentlichen quer zur Verschieberichtung der beweglichen Einrichtung (35) zwischen einer Ruheposition, in der die Ausgabeeinrichtung (30) nicht betätigt ist, und einer Betätigungspositionierung, in der die Ausgabeeinrichtung (30) betätigt ist, beweglich ist, wobei der Befestigungsring (60) einen oberen radialen Flansch (61) aufweist, der sich radial nach außen erstreckt, **dadurch gekennzeichnet, dass** das Betätigungselement (41) mindestens einen radialen Vorsprung (412) umfasst, von dem bei Betätigung ein oberer Rand (413) mit einem unteren Rand des radialen Flansches (61) in Kontakt tritt.

2. Vorrichtung nach Anspruch 1, wobei der Körper (10) mindestens eine Führungsfläche (50) des Betätigungselements (41) umfasst, wobei die mindestens eine Führungsfläche (50) in Bezug auf den Ausgabekopf (100) fest steht und im Wesentlichen parallel zur Verschiebeachse der beweglichen Einrichtung (35) in dem Ausgabeeinrichtungskörper (31) bei Betätigung der Ausgabeeinrichtung (30) ist, wobei das Betätigungselement (41) mindestens eine, vorzugsweise abgerundete, Endkante (411) umfasst, die mit der mindestens einen Führungsfläche (50) zusammenwirkt.

3. Vorrichtung nach Anspruch 2, wobei das Betätigungselement (41) mindestens eine Flanke (410) umfasst, die sich im Wesentlichen von der Drehachse (B) des Betätigungselements (41) bis zur Führungsfläche (50) erstreckt, wobei die Endkante (411) der Flanke (410) abgerundet ist und bei Betätigung auf der Führungsfläche (50) gleitet, wobei jede Flanke (410) einen radial vorstehenden Vorsprung (412) umfasst, der sich auf einer Seite der Flanke (410) in der Nähe seiner Endkante (411) erstreckt, wobei der radial vorstehende Vorsprung (412) eine obere, vorzugsweise abgerundete, Kante (413) aufweist, die mit dem radialen Flansch (61) des Befestigungsrings (60) zusammenwirkt.

4. Vorrichtung nach Anspruch 3, wobei das Betätigungselement (41) zwei Flanken (410) umfasst, die sich parallel zueinander erstrecken, wobei die radial vorstehenden Vorsprünge (412) einander gegenüber stehen.

5. Vorrichtung nach einem der vorhergehenden Ansprüche, wobei der Befestigungsring (60) einen ersten Befestigungsteil, der mit dem Behälter (20) zusammenwirkt, um die Befestigung des Befestigungsrings (60) an dem Behälter (20) zu gewährleisten, einen zweiten Teil, der mit der Ausgabeeinrichtung (30) zusammenwirkt, um die Befestigung der Ausgabeeinrichtung (30) in dem Befestigungsring (60) zu gewährleisten, und einen dritten Teil umfasst, der den oberen radialen Flansch (61) umfasst.

6. Vorrichtung nach Anspruch 5, wobei der obere radiale Flansch (61) mit einer axialen Hülse (63) verbunden ist, wobei der mindestens eine radiale Vorsprung (412) des Betätigungselements (41) außen an der axialen Hülse (63) angeordnet ist, wobei der obere Rand (413) des radialen Vorsprungs (412) bei Betätigung mit dem radialen Flansch (61) in Kontakt ist.

7. Vorrichtung nach einem der vorhergehenden Ansprüche, wobei das seitliche Betätigungssystem (40) ein Drückelement (42) umfasst, welches schwenkbar um eine Achse (A) auf dem Körper (10) montiert ist, wobei das Betätigungselement (41) schwenkbar um eine Achse (B) auf dem Drückelement (42) montiert ist.

8. Vorrichtung nach Anspruch 7, wobei das Betätigungselement (41) ein elastisches Element (415), wie ein elastisches Blatt, umfasst, das mit dem Drückelement (42) zusammenwirkt, um das Betätigungselement im Abstand von dem Drückelement (42) zu belasten.

## Claims

1. A fluid dispenser device comprising a body (10), a reservoir (20), a dispenser head (100) incorporating a dispenser orifice (5), and a dispenser member (30), such as a pump or a valve, that is mounted on said reservoir (20) by means of a fastener ring (60), said dispenser member (30) comprising a dispenser-member body (31) and a movable member (35) that moves axially in said dispenser-member body while said dispenser member (30) is being actuated, said device further comprising a lateral actuator system (40) that is secured to said body (10), said system including an actuator element (41) that co-operates with said fastener ring (60), said actuator element (41) being movable, substantially transversally to the direction of movement of said movable member (35), between a rest position in which said dispenser member (30) is not actuated, and an actuated position in which said dispenser member (30) is actuated, said fastener ring (60) including a top radial flange (61) that extends radially outwards, **characterized in that** said actuator element (41) includes at least one radial projection (412) having a top edge (413) that comes into contact with a bottom edge of said radial flange (61) during actuation.

2. A device according to claim 1, wherein said body (10) includes at least one guide surface (50) for guiding said actuator element (41), said at least one guide surface (50) being stationary relative to said dispenser head (100) and substantially parallel to the movement axis of the movable member (35) in the dispenser-member body (31) while said dispenser member (30) is being actuated, said actuator element (41) including at least one end edge (411) that is preferably rounded and that co-operates with said at least one guide surface (50).

3. A device according to claim 2, wherein said actuator element (41) includes at least one flank (410) that extends substantially from the pivot axis (B) of said actuator element (41) to said guide surface (50), the end edge (411) of said flank (410) being rounded and sliding over said guide surface (50) during actuation, each flank (410) including a radial projection (412) that extends on one side of said flank (410) in the proximity of its end edge (411), said radial projection (412) having a top edge (413) that is preferable rounded and that co-operates with said radial flange (61) of said fastener ring (60).

4. A device according to claim 3, wherein said actuator element (41) includes two flanks (410) that extend parallel to each other, said radial projections (412) facing each other.

5. A device according to any preceding claim, wherein said fastener ring (60) includes a first fastener portion that co-operates with the reservoir (20) so as to fasten said fastener ring (60) on said reservoir (20), a second portion that co-operates with the dispenser member (30) so as to fasten said dispenser member (30) in said fastener ring (60), and a third portion that includes said top radial flange (61).

6. A device according to claim 5, wherein said top radial flange (61) is connected to an axial sleeve (63), said at least one radial projection (412) of the actuator element (41) being arranged on the outside of said axial sleeve (63), with the top edge (413) of said radial projection (412) in contact with said radial flange (61) during actuation.

7. A device according to any preceding claim, wherein said lateral actuator system (40) includes a presser element (42) that is mounted on the body (10) to pivot about an axis (A), said actuator element (41) being mounted on said presser element (42) to pivot about an axis (B).

8. A device according to claim 7, wherein said actuator element (41) includes a resilient element (415), such as a resilient blade, that co-operates with said presser element (42) so as to urge said actuator element away from said presser element (42).
